# EUROPEAN PATENT APPLICATION

(11) **EP 0 992 793 A2**
(43) Date of publication of application: **12.04.2000**
(21) Application number: 99119637.9
(22) Date of filing: 04.10.1999
(51) Int. Cl.: G01N 33/50

(54) **Screening organic compounds for cognitive function modulating activity**

(30) Priority: 06.10.1998 US 167187; 01.04.1999 US 283553
(71) Applicant: Novartis AG, 4058 Basel (CH); Novartis-Erfindungen Verwaltungsgesellschaft m.b.H., 1230 Wien (AT)
(72) Inventor: Matus, Andrew Ian, 4104 Oberwil (CH); Kaech Petrie, Stefanie, 4056 Basel (CH); Fischer, Maria, 4052 Basel (CH)
(74) Representative: Becker, Konrad

(57) **Abstract**

The present invention provides a method for screening compounds which potentially modulate cognitive function comprising assaying for a modification of actin-dependent motility in response to a candidate compound and choosing a compound which modifies actin-dependent motility. More particular, the invention relates to method for screening compounds which potentially modulate cognitive function which includes comparing actin-based motility in cells displaying actin-dependent motility in the absence and the presence of the candidate compound and choosing a compound that modifies actin-dependent motility in said cells. Further, this invention relates to compounds which potentially modulate cognitive function and their medical and non-medical uses.

## Description

### Field of the invention

The present invention relates to the fields of neurobiology, pharmacology and medicine. It provides a method for screening compounds which potentially modulate cognitive function comprising assaying for a modification of actin-dependent motility in response to a candidate compound and choosing a compound which modifies actin-dependent motility. More particular, the invention relates to a method for screening compounds which potentially modulate cognitive function which includes comparing actin-based motility in cells displaying actin-dependent motility in the absence and the presence of a candidate compound and choosing a compound that modifies actin-dependent motility in said cells. Further, this invention relates to compounds which potentially modulate cognitive function and their medical and non-medical uses.

### Background of the invention

A wide variety of ideas about the function of dendritic spines have been proposed. These include that they exist solely to increase the surface area of the neuron available for receiving synaptic contacts, that their role is to protect neurons from excessive excitation, that they act as electrophysiological compartments independent of the dendrite or as biochemical compartments. Evidence that the numbers or shapes of dendritic spines can change according to the sensory input of animals during development or with an animal's behavioural status has led to the proposal that they may be involved in learning and memory. These hypotheses emphasize changes over hours or days. Recently it has been found that spines change shape rapidly over a period of seconds by a mechanism that is driven by the polymerization of actin (Fischer et al., Neuron, Vol. 20, 847-854, 1998; herein incorporated by reference in its entire content).

Volatile anaesthetics are thought to exert their effects by acting on proteins situated at central synapses. Among their potential targets most attention has focused on neurotransmitter receptors, several of which have been shown to be potentiated or inhibited by these agents. Recent studies using molecular genetic techniques further suggest that volatile anaesthetics act by binding to hydrophobic pockets in receptor proteins and several anaesthetic-sensitive sites with distinct molecular structures have been identified on different transmitter receptors. However, the mechanisms by which volatile anaesthetics exert their cognition-decreasing effects in the brain have not yet been elucidated. For example, little is known about the pathways and effector mechanisms that are involved. The lack of knowledge about these mechanisms is a severe problem in the identification of novel anaesthetics, as well as drugs that potentially modulate cognitive function in the human brain in general. Thus, there remains a need for novel assays for the identification of compounds for the modification of cognitive functions. In addition, there is a strong need to identify novel parameters whose modification by the application of compounds correlates with the modulation of cognitive functions in the human brain.

Unexpectedly, it now has been found that volatile anaesthetics block the spontaneous motility of spines at concentrations relevant to their clinical use. Surprisingly it is found that volatile anaesthetics also block actin-dependent motility in non-neuronal cell types such as fibroblasts. These findings identify the actin cytoskeleton itself is a direct or indirect target for anaesthetics and imply that the actin cytoskeleton in dendritic spines is involved in the maintenance of cognitive function in the brain and the conscious state.

In another surprising aspect of the invention it has been found that dendritic spine motility is inhibited by the activation of glutamate receptors, in particular ionotropic glutamate receptors e.g. glutamate receptors of the AMPA subtype, thereby identifying these receptors as potential targets for drugs modulating the maintenance of cognitive function in the brain and the conscious state.

### Detailed description of the invention

The present invention provides a previously unidentified link between the cognitive function of the brain and actin-dependent motility of dendritic spines. In particular, this invention identifies the actin cytoskeleton in dendritic spines as a novel target of volatile anaesthetics additional to the neurotransmitter receptors that have previously been identified as targets of these agents. Spines are particularly prevalent on major relay neurons in parts of the brain involved in cognitive function where they have been estimated to occur at between 70 and 90% of excitatory synapses. This distribution, together with their strategic location as bridges between the axon terminal and the dendrite, has led to the suggestion that changes in spine plasticity may be involved in learning and memory. The recent discovery that actin-based changes in spine shape can be detected in images taken only seconds apart suggests a connection with more rapid events in brain function. The surprising finding of the susceptibility of the actin cytoskeleton to volatile anaesthetics now identifies a relationship between spine dynamics and the maintenance of consciousness.

Accordingly, the present invention provides a method for screening compounds which potentially modulate cognitive function comprising assaying for a modification of actin-dependent motility in response to a candidate compound and choosing a compound which modifies actin-dependent motility.

The term "cognitive function" for the purposes of the present invention in particular includes cortical functions of the brain. Consciousness and memory therefore are examples of "cognitive function". Various conditions in the human, in particular diseases, may affect cognitive functions. For example, dementias such as Alzheimers disease or vascular dementia are characterized by an impaired cognitive function. Numerous diseases affecting the brain may affect cognitive function, in particular, diseases that affect the brain cortex are very likely to adversely affect cognitive function. Numerous tests to assess cognitive function are known to the person skilled in the art of psychology and medicine. For example, cognitive function may be assessed using tests known to a person skilled in the art such as standardized psychometric tests (e.g. Wechsler Memory Scale, the Wechsler Adult Intelligence Scale, Raven's Standard Progressive Matrices, Schaie-Thurstone Adult Mental Abilities Test), neuropsychological tests (e.g. Luria-Nebraska), metacognitive self-evaluations (e.g. Metamemory Questionnaire), visual-spatial screening tests (e.g. Poppelreuter's Figures, Clock Recognition, Honeycomb Drawing and Cancellation), cognitive screening tests (e.g. Folstein's Mini Mental State Test) and reaction time tests. Such standardized tests as listed above are described in Ruoppila,I. and Suutama,T. (1997) Scand. J. Soc. Med. Suppl. 53, 44-65 and serve as examples, said reference is incorporated in its entirety herein. The term "cognitive function" includes the functions assessed by any such test. The term "modulation" of cognitive function is meant to include increasing as well as reducing cognitive function. Increase as well as reduction of cognitive function may be assessed by any of the above-mentioned tests. In particular, in the sense of this invention it is understood that cognitive function is decreased in general to anaesthesia.

The term "actin-dependent motility" for the purposes of this invention includes movements by cells or parts of cells caused by the operation of actin protein either alone through its polymerization or in conjunction with other proteins such as e.g. myosin or cofilin. "Parts of cells" in the meaning of this invention include in particular protrusions of the cell membrane. Examples are dendrites, spines of dendrites of neurons and membrane ruffles as seen in example with fibroblasts. Thus, the term "modification of actin-dependent motility" includes modification of neuronal spine shape as well as modification of motility of fibroblasts. This invention accordingly also includes methods, wherein modification of actin-based motility is assessed as modification of neuronal spine shape as well as methods, wherein modification of actin-based motility is assessed as modification of motility of fibroblasts.

The term "modification" of actin-dependent motility includes reduction as well as increase of actin-dependent motility. Thus, the present invention comprises a method wherein a compound is chosen that reduces actin-dependent motility as well as a method wherein a compound is chosen that increases actin-dependent motility. The term "modification of actin-dependent motility" further includes modifications that are achieved either by direct molecular actions on actin or by indirect molecular actions on actin.

In one embodiment the present invention provides a method for screening compounds which potentially modulate cognitive function comprising the steps of: (a) measuring actin-dependent motility in suitable cells displaying such actin-dependent motility, (b) exposing such cells to a candidate compound, (c) measuring the actin-dependent motility in said cells in the presence of the candidate compound, (d) comparing actin-based motility in said cells in the absence and the presence of the candidate compound, (e) choosing a compound that modifies actin-dependent motility in said cells.

For example, the shape of a dendritic spine on a rat hippocampal neuron in culture may be visualized by a fluorescent protein (e.g. GFP-actin) enriched in the spine head and may be recorded at certain fixed intervals (e.g. every 10 sec) over a certain period of time (e.g. 5 min) prior to perfusion with a compound (e.g. Isoflurane at clinically relevant concentrations) in a solvent (e.g.saline) followed by an additional time-lapse recording over certain period of time after perfusion with said compound. The original recordings then may be overlayed by outlines generated using an image analysis software. Representations of the measured dimensions of the spine heads may be used for the calculation of the shape factor. For example, morphometric analysis of spine shape using the integrated morphometry analysis interface in Metamorph 3.0. may be employed (for further detail see below). Variations of the calculated shape factors before and after perfusion with the compound may then be compared and the compound may be chosen that lead to a change in the variability of the shape factors.

As a marker of dendritic spines GFP-actin may be used. For example, primary rat hippocampal neurons transfected with a GFP-tagged γ-cytoplasmic actin expression vector develop normally and acquire numerous spines in culture. GFP-actin targets and enriches in spine heads which are apposed by presynaptic terminals that may be visualized e.g. by immunofluorescence against an pre-synaptic vesicle marker, e.g. synaptophysin.

Video sequences may be used to directly demonstrate changes in actin-dependent motility. For example, a time-lapse recording of several spines on a dendritic segment of a GFP-actin transfected hippocampal neuron 24 days in culture over 5 min periods demonstrates constant change shape prior to the perfusion of an anaesthetic and immobility in presence of isoflurane. In addition, it demonstrates recovery of their motility after washout of isoflurane.

"Suitable cells" displaying actin-dependent motility for the purposes of the present invention are for example neuronal cells, cells of neuronal origin or fibroblasts. However, other non-neuronal cell types displaying actin-dependent motility may be suitable cells for the purposes of the present invention. The present invention therefore encompasses a method wherein the actin-dependent motility is actin-dependent spine motility as well as a method wherein the actin-dependent motility is actin-dependent cell motility.

Neuronal cells include cells derived from nervous tissue which have processes. Such processes are e.g. neurites and, in particular, axons or dendrites. Cells of neuronal origin include cells derived from nervous tissue, or their precursors such as the neural crest, either as primary neurons of, for example, the hippocampus, or as tumor cell lines, such as PC12 or NB2a, which possess aspects of the neuronal phenotype including especially the presence of long extensions of cell body, neurites, which are the characteristic features of neurons.

As above-mentioned, non-neuronal cells may also be employed for the purposes of this invention. For example, resting layers of rat embryo fibroblasts in saline may be treated for a certain periond of time (e.g.15 min) with 100 nM TPA which induces membrane ruffling. Cells may be fixed and stained e.g. with rhodamine-labelled phalloidin to visualize actin enrichment in ruffles at the edges of the cells. For example, pretreatment of cells with isoflurane prevents TPA-induced actin ruffles as may be demonstrated by the absence of bright staining at cell edges.

It is another aspect of this invention that actin-dependent motility can be inhibited in a dose-dependent fashion. It is a further aspect of this invention that actin-dependent motility can be inhibited in a reversible fashion. Accordingly, the present invention also provides a method wherein a compound is chosen that modifies actin-dependent motility in a dose-dependent fashion as well as a method wherein a compound is chosen that modifies actin-dependent motility in a reversible fashion. In a preferred embodiment the dose-dependency is "steep" in the sense that in a certain range of concentration a small change in concentration will lead to a relatively big change in actin-dependent motility. For example, effects of volatile anaesthetics actin-dependent motility reverse quickly. This may be demonstrated by showing that while the shape factor is constant in the presence of the anaesthetic, it varies over time after washout indicating changes in spine shape.

In a further embodiment of the present invention modification of actin-based motility is assessed by a method that includes visualization of actin filaments. In a preferred embodiment the method includes visualization of actin filaments with the help of cells expressing a fusion protein consisting of actin fused to a fluorescent molecule, which in a more preferred embodiment is a fluorescent protein. A fluorescent protein in the sense of the invention is a protein that emits light at one wavelength when stimulated by absorbing light at a second, exciting, wavelength. Examples are the green fluorescent protein derived from the jellyfish *Aequorea victoria* and artificial mutant forms produced by gene technology. In a preferred embodiment the present invention employs green fluorescent protein as the fluorescent molecule.

Further within the scope of this invention is a method of the invention wherein the candidate compound modifies the activity of glutamate receptors, in particular ionotropic glutamate receptors. In a preferred embodiment of the invention the candidate compound modifies the activity of glutamate receptors of the AMPA subtype. In another preferred embodiment of the invention the candidate compound inhibits the activity of glutamate receptors of the AMPA subtype.

The present invention also relates to a compound which has been identified by an assay according to this invention. Accordingly it includes such a compound which is a modulator of actin-dependent spine motility, in particular, an activator or an inhibitor of actin-dependent spine motility. Such compounds may e.g. be activators or inhibitors of the AMPA class of glutamate receptors.

Pharmaceutical preparations wherein the active ingredient is a compound of the invention are also within the scope of this invention. The preparation of a pharmacological composition that contains active ingredients dissolved or dispersed therein is well understood in the art. Such compositions may be prepared as injectables, either as liquid solutions or suspensions. Solid forms suitable for solution or suspension in liquid prior to use can also be prepared. A preparation can also be emulsified, or formulated into suppositories, ointments, creams, dermal patches, or the like, depending on the desired route of administration.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof, including vegetable oils, propylene glycol, polyethylene glycol and benzyl alcohol (for injection or liquid preparations); and petrolatum, vegetable oil, animal fat and polyethylene glycol (for externally applicable preparations). In addition, if desired, the composition can contain wetting or emulsifying agents, isotonic agents, dissolution promoting agents, stabilizers, colorants, antiseptic agents, soothing agents and the like additives (as usual auxiliary additives to pharmaceutical preparations), pH buffering agents and the like which enhance the effectiveness of the active ingredient.

The therapeutic compositions of the present invention can include pharmaceutically acceptable salts of the components therein. Pharmaceutically acceptable salts include the acid addition salts (e.g. formed with free amino groups) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a subject, e.g., a mammal, without the production of undesirable physiological effects such as nausea, dizziness, gastric upset and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin, vegetable oils such as cottonseed oil, and water-oil emulsions.

Further included in this invention is a method of decreasing cognitive function of the brain with an effective amount of such a compound, as well as a method of activating cognitive function of the brain with an effective amount of such a compound. Such a method may be employed for medical as well as non-medical purposes. For example, a method of treatment of a disease displaying enhanced cognitive function of the brain with an effective amount of a compound according to this invention that decreases cognitive function is included in this invention. Examples of such diseases are deliria, positive psychotic symptoms or hyperactivity syndromes. In addition, a method of treatment of a disease displaying impaired cognitive function of the brain with an effective amount of a compound according to this invention that increases cognitive function is part of this invention. Diseases displaying impaired cognitive function of the brain are numerous, e.g. dementias of the Alzheimer or Non-Alzheimer type, vascular strokes, benign and malign tumors or injuries affecting the cortex of the human brain. Essentially every disease that affects the human brain may also lead to a modulation of cognitive function in the sense of this invention.

Further, the use of a compound according to the invention that decreases cognitive function as an anaesthetic medicament is a preferred embodiment of the invention. Further preferred is a general anaesthetic medicament, particularly preferred is a volatile anaesthetic medicament. Uses of compounds as a sedative medicaments are also preferred embodiments and within the scope of this invention.

Non-medical purposes of such compounds are also contemplated by this invention. Examples are compounds of the invention that increase cognition in a mild to moderate fashion and may be consumed on a daily basis, i.e. for the enhancement of performance. The term "performance" for the purposes of this invention includes e.g. the learning and the memory functions of the brain. Other examples are compounds of the invention that decrease cognition in a mild to moderate fashion and may be helpful e.g. in relaxation and finding sleep.

### Examples

The invention is further described below, for the purpose of illustration only, in the following examples.

### Example 1

To examine the influence of volatile anaesthetics on spine motility their effects on live, cultured hippocampal neurons that have been transfected with green fluorescent protein-tagged actin (GFP-actin) to render their dendritic spines visible are studied. Video time-lapse recordings show potent inhibition of actin-based motility in dendritic spines when several chemically different volatile anaesthetics are applied. The nature of this effect is demonstrated for isoflurane in Table 1 which shows the variation of the shape factor calculated from a series of computer-generated profiles derived from individual frames taken from a video time-lapse recording of a single dendritic spine taken before and after the addition of isoflurane.
The following example further illustrates the employment of an assay according to the invention. First, the shape of a dendritic spine on a rat hippocampal neuron in culture as visualized by GFP-actin enriched in the spine head is recorded about every 5 sec over a period of about 6 min prior to perfusion with isoflurane. Then the cells are slowly perfused with isoflurane at clinically relevant concentrations in saline. This step is followed by an additional time-lapse recording over about 6 min. Then a morphometric analysis of spine shape is done using the integrated morphometry analysis interface in Metamorph 3.0. In particular, the shape factor calculated from the perimeter and the area of the spine illustrates changes in spine shape.
A comparison of the recorded shape factors revealed the following changes: Prior to the addition of the anaesthetics, the calculated shape factor changes dramatically over time, while in the presence of the isoflurane it remains constant. Thus, in this example isoflurane can be identified as a compound with the potential to modulate cognitive function, which indeed is known to be the case in the art of medicine. The described procedure may be employed in an analogous fashion to identify novel compounds that potentially modulate cognitive function.

Data for the entire experiment is shown in Table 1 where a shape factor calculated from the spine's shape is shown throughout the entire spine shape measurement. The wide distribution of values before addition of isoflurane indicates rapid variation in spine shape in contrast to the relative lack of change in this parameter in the presence of the anaesthetic.

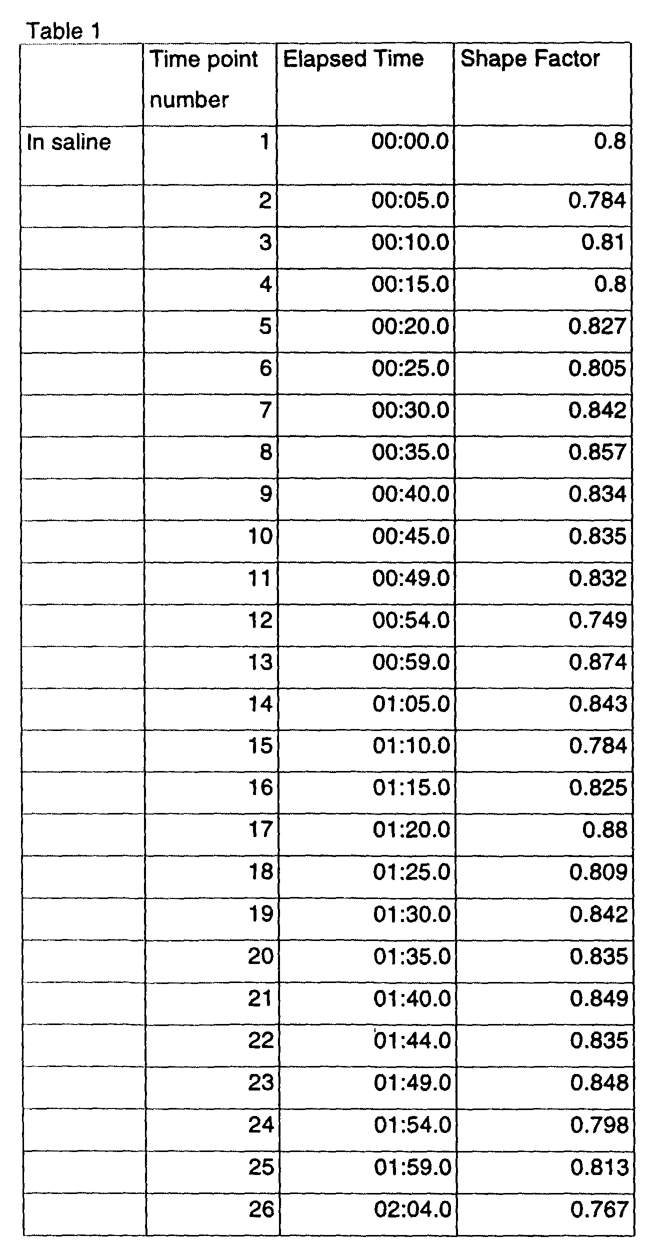

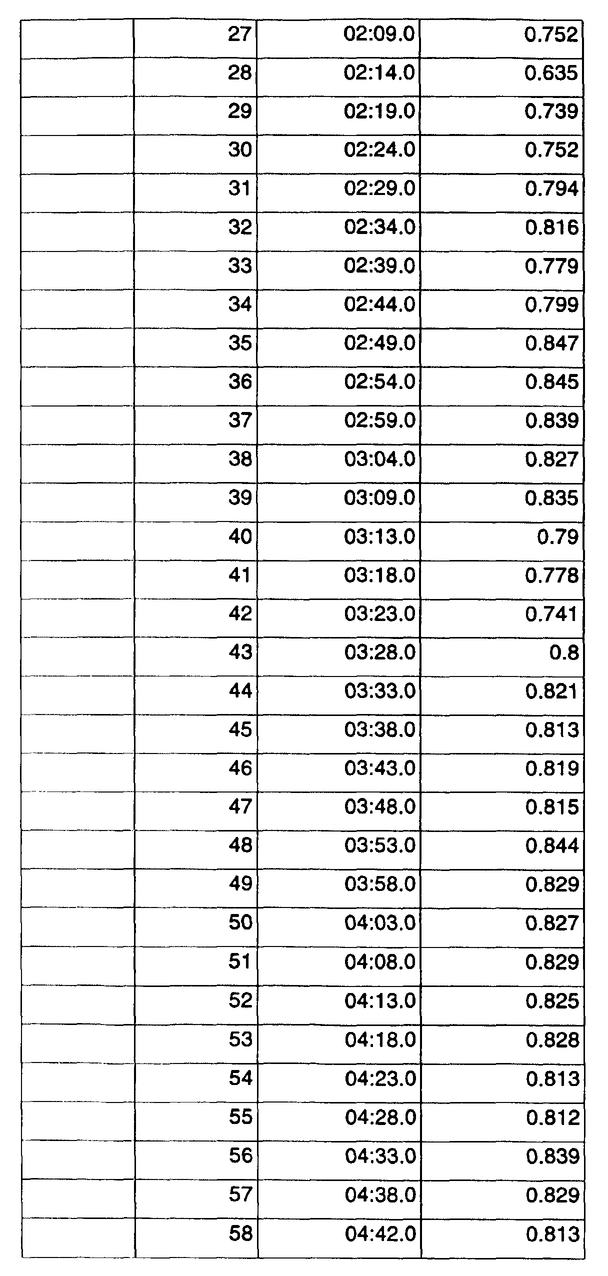

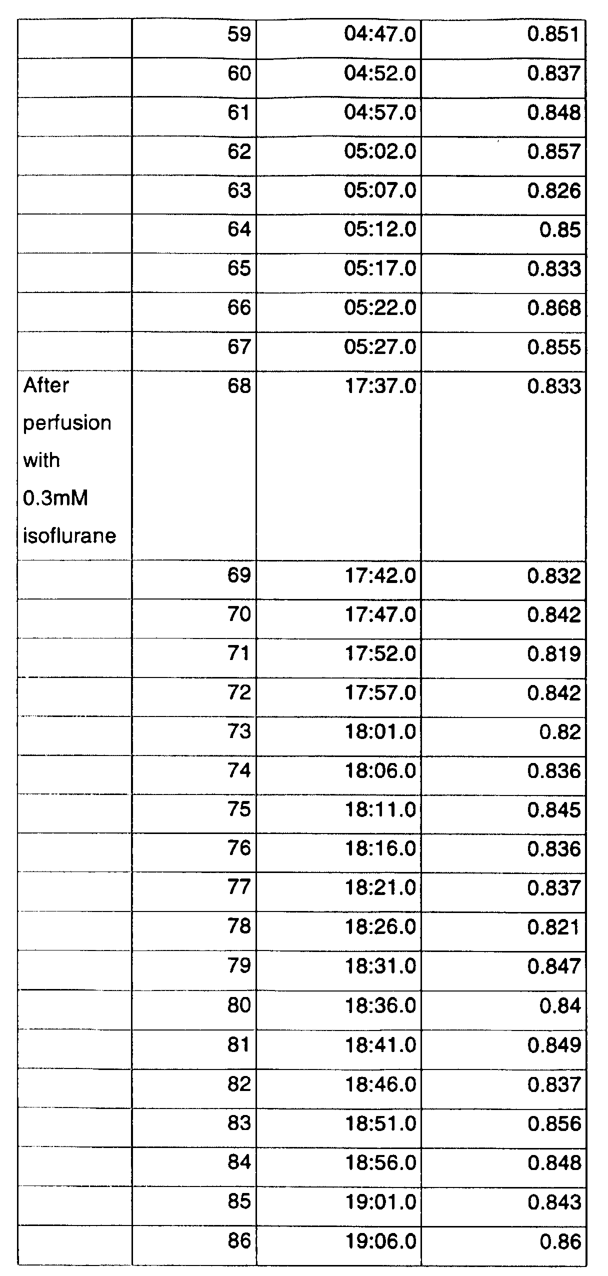

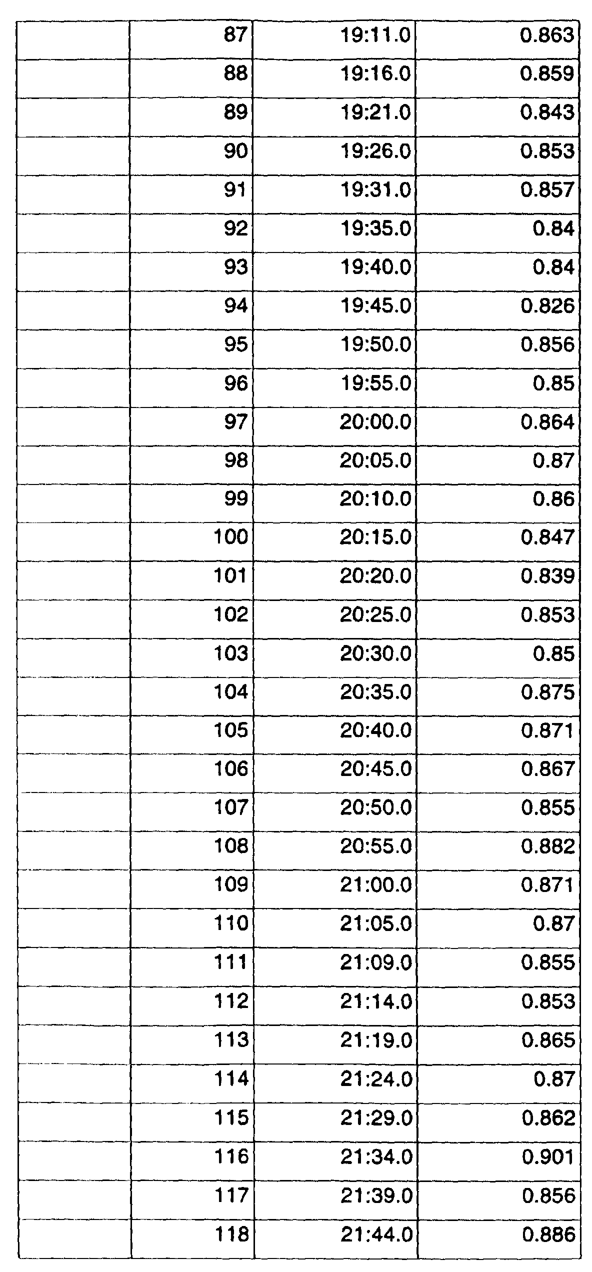

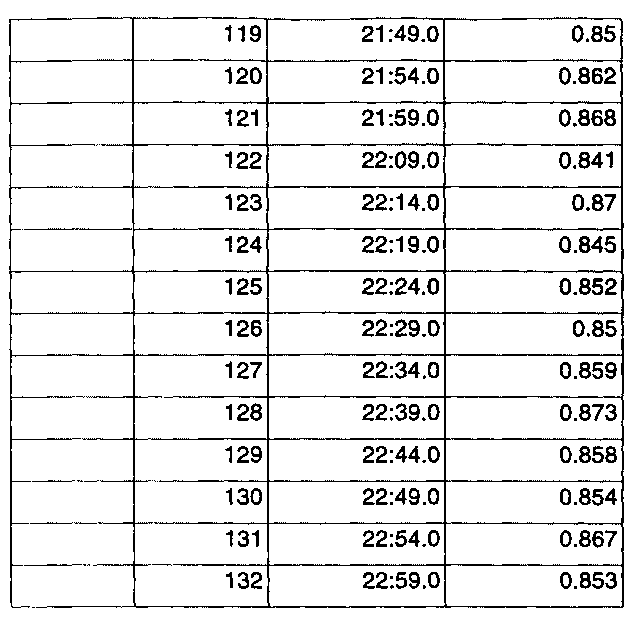

This inhibition of plasticity is not limited to individual spines but is generally evident on the dendrites of hippocampal neurons exposed to isoflurane. This can be appreciated by reference to the original time-lapse recording which also shows the recovery of motility when the anaesthetic is removed. As in previous experiments on spine motility the cultures used in these experiments are fixed and counterstained with antibodies against a presynaptic markers to confirm that spines are contacted by presynaptic terminals and are hence components of bone fide synapses.

### Example 2

Similar results to those for isoflurane are obtained with chloroform, enflurane, ether and halothane. Data obtained basically analagous as described in Example 1 for chloroform are presented in Table 2 which is arranged to show the recovery of spine motility when the anaesthetic is removed from treated cells. In the presence of chloroform variation in spine shape is minimal, while rapid changes in spine shape are resumed after chloroform is removed. Data for one spine over one entire recording is shown in Table 2.

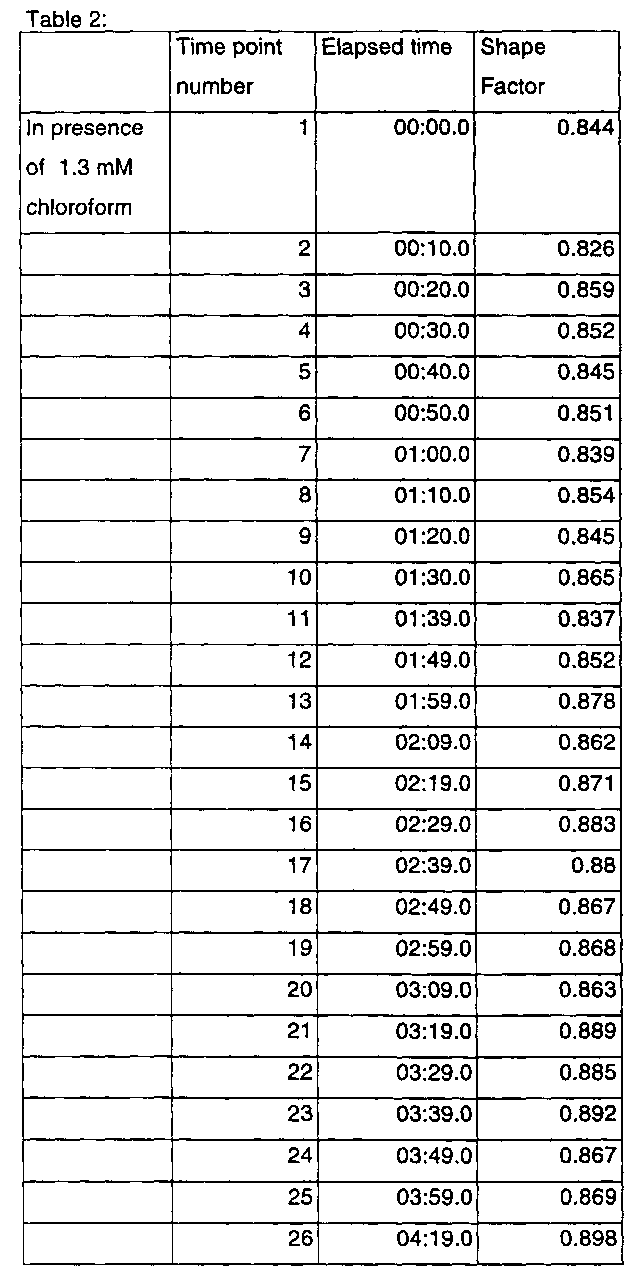

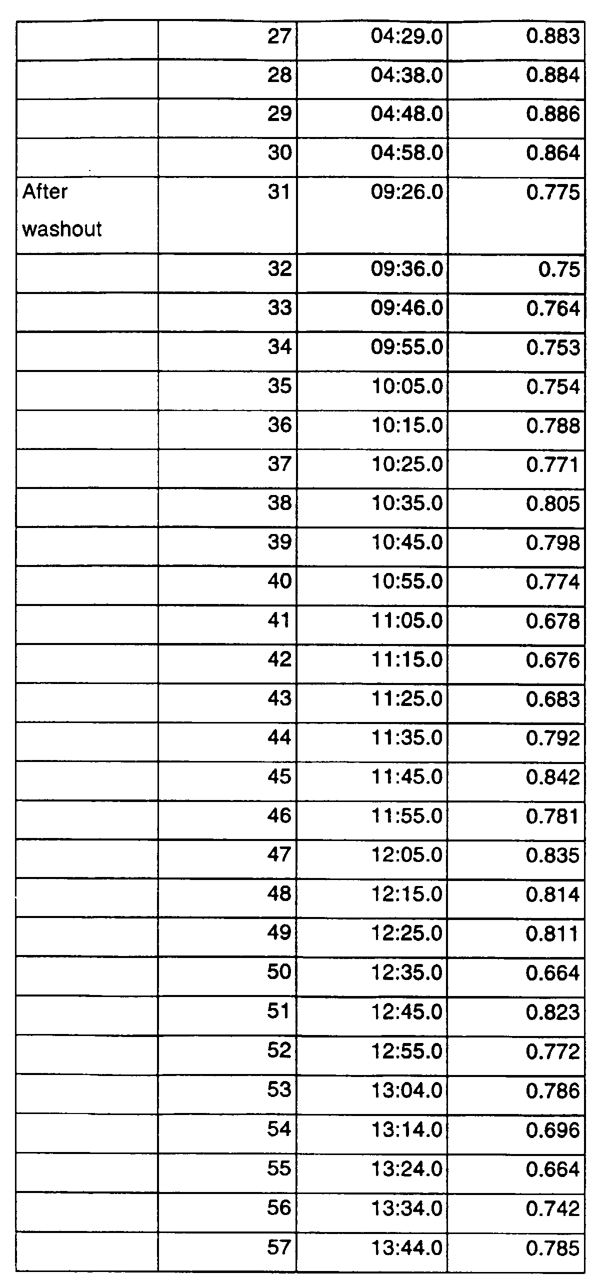

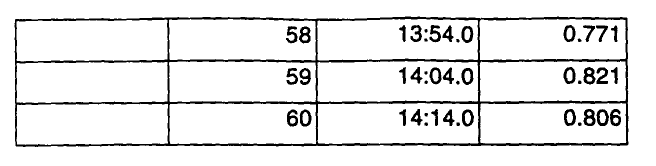

### Example 3

An important factor in judging the physiological relevance of biological effects induced by anaesthetics is the concentration at which they take place; relevant effects should occur at aqueous concentrations equivalent to the inhaled concentration required for anaesthesia. In this respect we find that the concentrations of these volatile agents needed to inhibit spine motility are closely similar to those that have been reported for their anaesthetic effects (Table 3).

**Table 3**

| Volatile | Effective conc. for spine motility (mM)¹ | Effective conc. for anaesthesia (mM)² |
|---|---|---|
| Chloroform | 1.33 | 1.30^{b} |
| Diethylether | 20.4 | 25.0^{b} |
| Enflurane | 0.30 | 0.64^{a} |
| Halothane | 0.23 | 0.27^{a} |
| Isoflurane | 0.30 | 0.31^{a} |

| | | |
|---|---|---|
| ¹ Calculated from concentration of saturated solution at 25°C used as stock solution. | | |
| ² Based on values for minimal alveolar concentration (MAC) in rats (^{a}) or EC₅₀ in tadpoles (^{b}). | | |

A further significant feature of the inhibition of spine motility by volatiles anaesthetics is its remarkably steep dose dependence: using isoflurane we observe that spines are fully motile at dilutions of 1:100 and 1:60 and are fully inhibited at a dilution of 1:50, corresponding in both steepness of dose response and effective concentration to the properties of this compound in anaesthesia.

### Example 4

The results of these experiments on neurons pose the question of whether volatile anaesthetics act directly on actin in dendritic spines or indirectly through neurotransmitter receptors, which are also influenced by these agents at clinically relevant concentrations, or some other component of central synapses. To choose between these possibilities the influence of volatile anaesthetics on actin-based motility is examined in fibroblastic cells, where neurotransmitter receptors and other elements of the synaptic machinery are absent. Each of the volatile anaesthetics tested strongly inhibits actin motility in rat embryo fibroblasts and does so at the same concentrations that are required for their effects on dendritic spine motility. Resting layers of rat embryo fibroblasts in saline are treated for 15 min with 100 nM TPA which induces membrane ruffling. Cells are fixed and stained with rhodamine-labelled phalloidin to visualize actin enrichment in ruffles at the edges of the cells. Pretreatment of cells with isoflurane prevents TPA-induced actin ruffles as demonstrated by the absence of bright staining at cell edges

### Example 5

Various evidence suggests that volatile anaesthetics act by binding to "hydrophobic pockets" in proteins leading us to consider the possibility that such a site, responsible for their potent inhibition of actin-based spine motility, might be present and associated with actin filaments, situated either within or between actin molecules. Since actin-based motility in dendritic spines depends on the actin polymerization cycle the possible influence of isoflurane on actin filament assembly is tested in vitro using a well-established pyrene-actin fluorescence assay. Isoflurane has no detectable effect on the polymerization state of pure actin, either during assembly or when added to pre-formed filaments indicating that, although the actin cytoskeleton is a target for volatile anaesthetics, the molecular site of action is not associated with actin itself.

### Example 6

In this example it is shown that dendritic spine motility is inhibited by AMPA. To assess the influence of glutamate receptors on dendritic spine motility video recordings are made during which agonists and antagonists of ionotropic glutamate receptors are transiently applied to hippocampal neurons expressing GFP-actin. For example, such a recording is made from part of the dendrite of a GFP-actin expressing cell in a culture that is exposed to 200nM AMPA. In initial titration experiments, AMPA is effective at concentrations as low as 50 nM. Substantial changes in spine shape occur prior to the application of AMPA (control), and after it is washed out (recovery), but during the period when AMPA is present, spine movement effectively ceases (centre panels). The striking extent of this motility blockade may be illustrated by video data. This effect of AMPA is mimicked by the application of Kainate and entirely blocked by the selective AMPA receptor antagonist 2-Amino-3-[3-(carboxymethoxy)-5-methyl-isoxazol-4-yl]propionic acid (AMOA). In order to exclude the effect being a consequence of indirect NMDA receptor activation, the experiment may be repeated in presence of the selective NMDA receptor antagonists APV and MK801. Data may be recorded for several spines on a single dendrite segment at high magnification so that the effect of AMPA on individual spines is visible. The frames from such a recording and the corresponding difference images show that blockade of NMDA receptors by MK801 does not inhibit spine motility and that the presence of MK801 does not influence the inhibition of motility by AMPA. The results for APV are equivalent. Such frames are taken from a longer video recording encompassing a much larger area of this dendrite and several different drug combinations. Recordings made with AMPA at concentrations that alter spine motility, 50 nM to 200 nM are reversible and do not produce dendritic varicosities which are associated with subsequent cell death.

### Example 7

In this example it is shown that activation of AMPA receptors leads to a change in spine shape and actin distribution. Beside of blocking spine motility, AMPA receptor activation induces consistent changes in spine shape. This may be illustrated by computer-generated profile images of spines before, during and after exposure to AMPA. As well as illustrating the absence of shape change when AMPA is present, the rounding up of the spine induced by receptor activation is evident. This is not an isolated case; e.g. of 152 spines randomly chosen from dendrites of 4 different cells for detailed examination AMPA-induced rounding is plainly evident in 140 (92%) of them. The association of this shape change with AMPA-induced blockade of spine motility can be judged form a corresponding video sequence. To obtain a measure of these AMPA-induced changes in spine shape a computer routine to calculate a shape factor for spines in successive frames from video recordings may be used. The shape factor compares the perimeter of an object (e.g. the spine head) with its area, providing a criterion of shape regularity: a value of 1 corresponds to an exactly round profile while values below this indicate increasing irregularity. The rounding up of the spines in response to AMPA is demonstrated by the values between 0.8 and 0.9 and the stability of this shape is shown by the lack of deviation from this value while AMPA is present. By constrast this value varies widely and rapidly in the absence of AMPA, consistent with the visible changes in spine shape. Recovery after removal of AMPA is very rapid (< 15 min); the shape factor is fluctuating as soon as we resumed recording after washing out the AMPA. Rounded spine heads after AMPA treatment may also be illustrated by immunohistochemical stainings. The spine heads, constituting the postsynaptic part of the excitatory synapse, are contacted by presynaptic terminals which may be indicated by the staining for the markers synaptotagmin and bassoon. They express the AMPA receptor subunit GluR2. Phase micrographs of spine heads protruding from the dendritic surface and not covered by axon bundles show that the spine head is completely filled with gfp-actin and that its outline denotes the outline of the spine.

### Example 8

In this example it is shown that AMPA treatment appears to involve a redistribution of spine actin. A further consistent consequence of treating hippocampal neurons with AMPA is a visible change in the distribution of GFP-actin fluorescence involving an apparent shift toward a concentration in the centre of the spine head. GFP-actin fluorescence intensity may be measured in successive frames of video recordings in which AMPA is transiently present. Measurements are made for a whole dendritic segment and within boxes of the same area placed in the center of spine heads or on the dendritic shaft, respectively. After application of AMPA, a decrease in intensity over the whole dendrite is observed. This phenomenon could reflect some general aspect of GFP fluorescence in response to an altered ion concentration or pH of the local cytoplasm. Fluorescent excitation of GFP in solution decreases at low pH. Therefore the observed decrease in brightness might be attributed to a lowered pH, mediated by glutamate receptor activation. Application of glutamate or Ca2+ ionophores to rat primary cultured hippocampal neurons is shown to induce a persistent acidosis. To account for this effect, data are normalized for single spines to the overall brightness of the dendrite. The average brightness from the boxes during the recordings for three randomly selected spines may then be evaluated. For all three spines a marked increase in GFP-actin fluorescent intensity in the centre of their heads is observed, while the average brightness of the dendritic shaft is reduced. For above mentioned reasons, it is not entirely clear, if the apparent reduction in spine diameter is due to loss of actin fluorescence because of pH shifts or actin concentration in the center of spine heads, but comparison of phase and GFP-actin pictures argue in favour of changes in spine head size. The same caution has to apply to changes in the diameter of the spine neck, which have been postulated to influence synaptic weight by altering the electrical resistance. Those structures are extremely small and for a detailed analysis electronmicroscopic analysis would be favourable.

### Methods

The entire content of all of the cited publications is incorporated herein by reference.

### Anaesthetics

Chloroform and diethylether are purchased from Sigma Chemicals/Fluka Biochemicals, Buchs, Switzerland. Isoflurane (Forene) and enflurane (Ethrane) are from Abbott Laboratories, Cham, Switzerland. Methoxyflurane, manufactured by Mallinkrodt, is purchased through Arovet, Zollikon, Switzerland and halothane is distributed by Research Biochemicals International, Buchs, Switzerland. All anaesthetics are applied from saturated solutions in phosphate-buffered saline. These saturated solutions have the following concentrations (McKenzie, D., Franks, N.P. and Lieb, W.R., British Journal of Pharmacology (1995) 115, 275-282): for chloroform 66 mM, for diethylether 816 mM, for methoxyflurane 9.1 mM, for isoflurane 15.3 mM and for enflurane 11.9 mM

### Cell culture

All reagents for cell culturing are obtained either from Life Technologies, Basel, Switzerland, or from Sigma Chemicals/Fluka Biochemicals, Buchs, Switzerland. Cultures of primary rat hippocampal neurons are established exactly according to Goslin and Banker (Rat hippocampal neurons in low-density culture, in *Culturing Nerve Cells* (Bradford Book, MIT Press, Cambridge, MA, 1991) G.Banker and K.Goslin, eds.). Briefly, dissected hippocampi of E19 rat embryos are digested with 0.25% trypsin for 15 min at 37°C and triturated to yield a single cell suspension using a narrow-bore pasteur pipet. Neurons are plated onto polylysine treated glass coverslips in Eagle's minimal essential medium supplemented with 10% horse serum. After a 4 hr incubation the coverslips are turned over onto confluent layers of primary astroglial feeder layers in chemically defined Eagle's minimal essential medium with N2 supplement. Over a time period of 3-4 weeks, neurons develop large networks of processes and eventually matured to carry synapses on dendritic spines. The rat embryo fibroblast cell line REF52, obtained from K. Ballmer (Paul Scherrer Institute, Wuerenlingen, Switzerland), is maintained in Dulbecco's modified Eagle's medium supplemented with 10% fetal calf serum.

### Constructs and transfection

The expression constructs encoding fusion proteins of gamma- or beta- cytoplasmic isoforms of actin with enhanced green fluorescent protein (taken from pEGFP-N1; Clontech, Palo Alto, CA, USA) under the control of a chicken beta-cytoplasmic actin promoter have been described (Fischer, M., Kaech, S., Knutti, D. and Matus, A., Neuron (1998) 20, 847-854).
Neurons are transfected during the isolation protocol using DOTAP (Roche Biochemicals/Boehringer Mannheim, Rotkreuz, Switzerland) essentially as described (Kaech , S., Kim, J. B., Cariola, M., and Ralston, E., Mol. Brain Res. (1996) 35, 344-348): 18 µl of DOTAP and 3 µg of plasmid DNA are incubated with 10⁶ freshly isolated neurons in 5 ml Hank's buffered saline for 45 min at 37°C prior to plating on coverslips.

### Microscopy and Image analysis

For live imaging, coverslips are mounted in saline in closed observation chambers (Type 1, Life Imaging Services, Olten, Switzerland ) and placed at 37°C on a DM-IRBE inverted fluorescence microscope (Leica, Basel, Switzerland) equipped with high numerical aperture oil lenses and a GFP-optimized filter set (Chroma Technologies, Brattleboro, VT, USA). Images are captured using a cooled CCD camera (Princeton Instruments, Trenton, NJ, USA) and MetaMorph Imaging Software version 3.0 (Universal Imaging Corporation, West Chester, PA, USA). Anaesthetics are diluted from saturated solutions in saline into prewarmed saline immediately prior to slow perfusion into the closed observation chambers at a rate of approx. 0.5ml/min for a total exchange of 5 ml volume.
For analysis of spine shape over time the integrated morphometric function of MetaMorph 3.0, in particular the shape factor of thresholded objects calculated from their perimeter and their area, is used to log the data for individual images directly to Microsoft Excel 97.
For analysis of the actin cytoskeleton in REF52, cells plated on glass coverlips are serum-starved for several hours prior to the experiment. Coverslips are mounted in observation chambers to replicate the circumstances for perfusion with anaesthetics during live imaging.

Actin ruffling is induced by slow perfusion with 100 nM TPA (Alexis Biochemicals, Laeufelfingen, Switzerland) in phosphate-buffered saline either in the presence or absence of anaesthetics. Cells are then fixed with 4% paraformaldehyde (Fluka Biochemicals, Buchs, Switzerland) in phosphate-buffered saline with 0.02% Triton X-100 (Fluka Biochemicals, Buchs, Switzerland) and 10 µg/ml rhodamine-labelled phalloidin F(Sigma Chemicals, Buchs, Switzerland) for 10 min at room temperature, rinsed in phosphate-buffered saline and mounted in Vectashield (Vector Laboratories, Burlingame, CA,USA) for inspection on the fluorescence microscope.

### Transgenic animals

Transgenic animals are generated by injecting a 5.5 kb Ndel/EcoRI-fragment of a C-terminal GFP-tagged gamma-cytoplasmic actin construct into mouse oocytes of strain xy mice as described previously (Bottery et al., 1987, details and characterization see Wagner et al., in prep.). Animals are bred and analysed by Southern blots of genomic DNA prepared from tail clippings, PCR and direct inspection under a UV-Binocular microscope (Leica MZ12). The brains of mice expressing the construct under the control of the chicken β-actin promotor (Fregien and Davidson, 1986, Meichsner et al., 1993) are used to prepare organotypic slice cultures.

### Slice culture

Hippocampal slice cultures are prepared and maintained as described by Gahwiler (Gahwiler, B.H. 1981. Organotypic monolayer cultures of nervous tissue. J Neurosci Methods. 4:329-342. Gahwiler, B.H. 1981. Morphological differentiation of nerve cells in thin organotypic cultures derived from rat hippocampus and cerebellum. Proc R Soc Lond B Biol Sci. 211:287-290.). After 4 weeks, they are mounted in an observation chamber (Life Imaging Services, Olten, Switzerland) and constantly perfused with the imaging or test buffer (Tyrodes, as above or Tyrodes containing 1 mM AMPA, respectively). Filter sets for fluorescent microscopy are the same as for dispersed cultures. The Leica IRBE microscope is armed with an additional confocal head (CSU10, Yokogawa, Japan). Frames are acquired with an Astrocam CCD camera and UltraView imaging software (both LSR, Cambridge, UK).

### Application of drugs

Drugs are either applied by gravity feed perfusion or by removing the chamber lid and adding the test substance. Similar results are obtained for imaging in glia conditioned medium or in Tyrodes (contains glucose, Life Technologies, Basel), supplemented with 10 mM glycin and 0.2 g/l Na₂HPO₄ (to achieve a phosphate buffer for prolonged imaging without CO₂ gasing). The pH is adjusted to 7.2. [NMDA and AMPA receptor activity is influenced by the extracellular concentration of protons (Kashiwagi et al., 1997; Saybasili et al., 1998; Mc Donald et al., 1998)]

### Determination of the activity of glutamate receptors

L-Glutamate is the main excitatory neurotransmitter within the central nervous system. It conveys fast excitatory signals via ionatropic receptors which are ligand-gated ion channels. Ionotropic glutamate receptors (iGluRs) are composed of three classes of subtypes according to their selectivity for the agonists N-methyl-D-aspartate (NMDA), α-amino-3-hydroxy-5-methylisoxazole-4-propionic acid (AMPA) and kainate. Each subclass is further composed of various subunits that contribute to the receptor diversity (Watkins and Evans, 1981, Ann Rev. Pharmacol. Toxicol. 21, 165-204; Mayer and Westbrook, 1987, Prog. Neurobiol. 28, 197-276; Collingridge and Lester, 1989, Pharmacological Reviews 40/2: 143-210; Seeburg, 1993, Trends Neurosci. 16, 359-365)

The activity of glutamate receptors is assayed by adding appropriate pharmacological agents to the medium while recording the electrical activity of neurons. For AMPA receptors the activity is measured before and after the additon of AMPA at concentrations necessary to induce changes in spine motility, namely between 50 nM and 200 nM.

The ability of a drug to interfere with the glutamate transmission can be assessed in vitro in two ways. In a receptor binding type of assay, the direct affinity of a given drug for the receptor is assessed by measuring the capability to compete with a radiolabeled agonist/antagonist that binds specifically to the receptor. This approach gives indications for affinity/potency and specificity versus other bindings sites or receptors. However, to distinguish agonistic from antagonistic properties, an in vitro functional assay is required. The electrophysiological measurement of neuronal activity is most suited to distinguish between the ability of a drug to depolarize neurons as glutamate (agonist) or to inhibit the excitatory effects of glutamate or specific agonists (antagonist).

The interaction of a drug with a given site of the NMDA receptor complex or the AMPA or kainate receptor is studied by using specific ligand. In case of the NMDA receptor complex, [³H]CGP 39653 is used to label the competitive NMDA/Glu site, (Sills et al, 1991, Eur. J. Pharmacol. 192, 19-24), [³H]MDL105,519, or [³H]DCKA the modulatory glycine binding site (Baron et al, 1991, Eur. J. Pharmacol. 206, 149-154), and [³H]MK-801 to label the non competitive channel site (Ransom & Stec, 1988, J. Neurochem. 51, 830-836), Interaction with the AMPA and kainate receptors are examined by using [³H]AMPA (Honore et al, 1982, J. Neurochem. 38, 173-178) and [³H] kainate (Simon et al, 1976, J. Neurochem. 26, 141-147) respectively.

The radioligand binding assays are performed on semi-purified rat brain membrane preparations. Nonspecific binding is, in each case measured with access of glutamate, or glycine or MK 801.

The electrophysiological experiments are performed on rat cortical slices. In these preparations, NMDA receptors mediate relatively slow and highly voltage dependent excitatory postsynergetic potentials (EPSPs) or currents (EPSCs) whereas AMPA receptors mediate fast and voltage-independent currents (Collingridge and Walkins, 1994, The NMDA receptor, 2nd edition, Oxford University Press; Westbrook, 1994, Current Opinion in Neurobiology 4, 337-346). Since it has long been known that virually all cortical neurons are excited by L-glutamate and other excitatory amino acids Harrison and Simmonds (Harrison and Simmonds (1985) Br. J. Pharmacol. 84, pp381-391) described a preparation using rat neocortical slices which could be placed in a two-compartment chamber so that depolarization of many neurons could be recorded as a population response to these excitatory amino acids. Since then it has been exploited by many others primarily in the investigation of glutamate pharmacology (e.g. Burton et al., 1988, Br. J. Pharmacol. 93, pp693-701; Brugger et al., 1990, Eur. J. Pharmacol. 191, pp29-38; Lingenhöhl and Pozza, 1998, Neuropharmacology, 37, pp729-737) to either construct concentration response curves for agonistic and antagonistic actions of research compounds or to assess the selectivity of a research compound against depolarizations induced by NMDA, AMPA and kainate.

## Claims

1. A method for screening compounds which potentially modulate cognitive function comprising assaying for a modification of actin-dependent motility in response to a candidate compound and choosing a compound which modifies actin-dependent motility.

2. A method for screening compounds which potentially modulate cognitive function comprising the steps of: (a) measuring actin-dependent motility in suitable cells displaying such actin-dependent motility, (b) exposing such cells to a candidate compound, (c) measuring the actin-dependent motility in said cells in the presence of the candidate compound, (d) comparing actin-based motility in said cells in the absence and the presence of the candidate compound, and (e) choosing a compound that modifies actin-dependent motility in said cells.

3. A method according to claim 2 wherein the suitable cells displaying actin-dependent motility are selected from the group consisting of neuronal cells, cells of neuronal origin and fibroblasts.

4. A method according to claim 1 wherein a compound is chosen that reduces actin-dependent motility.

5. A method according to claim 1 wherein a compound is chosen that increases actin-dependent motility.

6. A method according to claim 1 wherein the actin-dependent motility is actin-dependent spine motility.

7. A method according to claim 1 wherein the actin-dependent motility is actin-dependent cell motility.

8. A method according to claim 1 wherein a compound is chosen that modifies actin-dependent motility in a dose-dependent fashion.

9. A method according to claim 1 wherein a compound is chosen that modifies actin-dependent motility in a reversible fashion.

10. A method according to claim 1 wherein modification of actin-based motility is assessed as modification of neuronal spine shape.

11. A method according to claim 1 wherein modification of actin-based motility is assessed as modification of motility of fibroblasts.

12. A method according to claim 1 wherein modification of actin-based motility is assessed by a method that includes visualization of actin filaments.

13. A method according to claim 2 wherein a compound is chosen that reduces actin-dependent motility.

14. A method according to claim 2 wherein a compound is chosen that increases actin-dependent motility.

15. A method according to claim 2 wherein the actin-dependent motility is actin-dependent spine motility.

16. A method according to claim 2 wherein the actin-dependent motility is actin-dependent cell motility.

17. A method according to claim 2 wherein a compound is chosen that modifies actin-dependent motility in a dose-dependent fashion.

18. A method according to claim 2 wherein a compound is chosen that modifies actin-dependent motility in a reversible fashion.

19. A method according to claim 2 wherein modification of actin-based motility is assessed as modification of neuronal spine shape.

20. A method according to claim 2 wherein modification of actin-based motility is assessed as modification of motility of fibroblasts.

21. A method according to claim 2 wherein modification of actin-based motility is assessed by a method that includes visualization of actin filaments.

22. A method according to claim 1 wherein the candidate compound is a compound which modifies the activity of glutamate receptors.

23. A method according to claim 1 wherein the candidate compound is a compound which modifies the activity of glutamate receptors of the AMPA subtype.

24. A method according to claim 2 wherein the candidate compound is a compound which modifies the activity of glutamate receptors.

25. A method according to claim 2 wherein the candidate compound is a compound which modifies the activity of glutamate receptors of the AMPA subtype.
